# EUROPEAN PATENT APPLICATION

(11) **EP 4 501 379 A1**
(43) Date of publication of application: **05.02.2025**
(21) Application number: 23189262.1
(22) Date of filing: 02.08.2023
(51) Int. Cl.: A61M 15/00

(54) **INHALER MEASUREMENT DEVICE**

(71) Applicant: Gerresheimer Werkzeugbau Wackersdorf GmbH, 92442 Wackersdorf (DE)
(72) Inventor: KRÜGER, Dirk, 48163 Münster (DE); KÄMEREIT, Markus, 48301 Nottuln (DE)
(74) Representative: Bardehle Pagenberg Partnerschaft mbB Patentanwälte Rechtsanwälte

(57) **Abstract**

The present disclosure is directed to an inhaler measurement device (1), in particular an inhaler flow measurement device, for a portable inhaler (100). The inhaler measurement device (1) comprises a housing (10) having a medication inhalation channel (11), wherein the medication inhalation channel (11) has a puff receiving end (12) being configured to receive a puff of spray (5) from the portable inhaler (100) and an inhalation end (13) being adapted to provide the puff of spray (5) to an individual. Further, the housing (10) comprises a flow measurement channel (15), wherein the flow measurement channel (15) has an air inlet (16), an air outlet (17), and a flow sensor (18) arranged between the air inlet (16) and the air outlet (17).

## Description

### 1. Technical field

The present disclosure relates to an inhaler measurement device and a measurement system comprising said inhaler measurement device.

### 2. Prior art

In the field of inhalers, and medical inhalers in particular, there is a need to ensure that an individual, e.g., a patient, inhales a substance, e.g., a medication, provided by the inhaler as completely as possible and/or receives a medication at a sufficient dose. Otherwise, depending on the case of use, there is a threat of health risks. Additionally, since numerous medications are in short supply and correspondingly difficult to obtain, there is also a need that an efficient use of the medication delivered by the inhaler is ensured.

To address these needs, a key aspect is to detect whether an individual has inhaled the substance/medication provided by the inhaler. In this context, the duration and/or intensity of inhalation may be decisive in determining whether an individual has received a substance in sufficient quantity or at all. To achieve this, various measurements can be carried out to determine whether, how intense and/or for how long an inhalation was conducted by the individual. Based on these measurements, it is regularly attempted to determine whether an individual has received the required medication and particularly if the individual has received the required medication at a sufficient dose. Furthermore, the measurement of the inhalation process should regularly determine whether inhalation has taken place with sufficient intensity and/or duration so that the delivered substance is used as efficiently as possible. However, the present measurement methods have several disadvantages, as explained below using specific examples.

A first variant of how measurements are performed on inhalers is the measurement of an airflow inside a medication inhalation channel of the inhaler. In this variant, however, sensors are regularly obstructed by components of the substance to be inhaled, so that falsified measurements occur. In addition, components of the substance to be inhaled regularly adhere to the sensors, so that a reduction of the substance provided to the individual occurs.

A second variant of how measurements are performed on portable inhalers is to measure an airflow in an area adjacent to a cartridge of the inhaler, as this is where the aspirated air regularly passes through. This variant is intended to provide information on whether an individual has inhaled and in particular if the individual has inhaled with sufficient intensity. However, this variant has the disadvantage that the flow in the area adjacent to the cartridge is regularly distorted by the finger that is used to trigger the inhaler.

Thus, one of the overall objectives of the present disclosure is to provide an inhaler measurement device and a respective measurement system that overcome the aforementioned drawbacks at least partially. Particularly, it is an overall objective of the present disclosure to provide an improved inhaler measurement device and an improved measurement system that allow a more accurate information on an individual's inhalation behavior and preferably a more accurate information on a substance delivery through the inhaler.

### 3. Summary of the invention

This objective is achieved, at least partly, by an inhaler measurement device and a measurement system, as defined in the independent claims. Further aspects of the present disclosure are defined in the dependent claims.

In particular, the objective is achieved by an inhaler measurement device, in particular an inhaler flow measurement device, for a portable inhaler. The inhaler measurement device comprises a housing having a medication inhalation channel. The medication inhalation channel has a puff receiving end being configured to receive a puff of spray from the portable inhaler and an inhalation end being adapted to provide the puff of spray to an individual. Moreover, the housing further comprises a flow measurement channel, wherein the flow measurement channel has an air inlet, an air outlet, and a flow sensor arranged between the air inlet and the air outlet.

The housing may comprise a plurality of individual housing parts. For example, the housing may comprise an inner housing structure that carries the puff detection device and optionally respective electrical components. Moreover, the housing may further comprise an outer circumferential sleeve which protects the inner housing structure and the respective components attached to it. Furthermore, the medication inhalation channel may be formed within a separate channel component of the housing, wherein preferably said channel component can be separated from the housing. By said channel component a cleaning and/or a replacement may be facilitated.

The medication inhalation channel preferably has an oval cross-section and/or a circular cross-section. It has been shown that these cross sections allow reduced adhesion of components of a puff of spray, for example, compared to cross sections with corners.

The puff receiving end may be defined as a receiving portion of the inhalation channel which is configured to receive a puff of spray from the portable inhaler. Hence, the puff receiving end may not necessarily correspond to a structural end of a channel. For example, if the inhaler measurement device has a channel for inserting a mouthpiece of an inhaler, then a region of the medication inhalation channel which is arranged to be adjacent to the end of said mouthpiece be referred to as the puff receiving end, i.e., the puff receiving portion. The inhalation end, which is adapted to provide the puff of spray to an individual, may be defined as an inhalation portion of the inhalation channel, where the puff of spray leaves the inhaler measurement device.

A puff of spray may be defined as a discrete and/or controlled release of a liquid and/or an aerosol from a preferably pressurized container, e.g., a pressurized Metered Dose Inhaler (pMDI), a cartridge, through a nozzle and/or a valve mechanism. The puff of spray may be propelled by a compressed gas and/or a mechanical means. The puff of spray may be characterized by its temperature, cloud cone, noise, speed, volume and/or duration. Said volume and/or duration may be determined by user interaction and/or the configuration of the portable inhaler.

The flow measurement channel may comprise a first channel portion being associated with the air inlet. Further, the flow measurement channel may comprise a second channel portion being associated with the air outlet.

The flow sensor may be configured to measure and/or monitor a differential pressure, a flow rate and/or a flow velocity of a fluid, e.g., air, passing through the flow measurement channel. Moreover, the flow sensor may provide quantitative information about the volume and/or mass of the fluid flowing through the flow measurement channel per unit of time. The flow sensor may comprise a differential pressure flow sensor, a turbine flow sensor, an ultrasonic flow sensor, a thermal flow sensor, and/or a vortex flow sensor. Said thermal flow sensor may be particularly preferred, as such sensors exist in small sizes. Thermal flow sensors utilize the principle of heat transfer to measure the flow rate. They have a heated element and temperature sensors. The flow of fluid affects the rate of heat dissipation, and by measuring the temperature difference, the flow rate can be determined.

The inhaler measurement device, according to the present disclosure, has several advantages, which will become apparent to the person skilled in the art when reading the following description. However, one specific advantage of the inhaler measurement device, according to the present disclosure, is that it provides a more accurate information on an individual's inhalation behavior, especially when compared to prior art solutions described in the introduction. Particularly, since the intensity and/or duration of an inhalation by an individual can be reliably detected by the flow sensor. Moreover, by the flow measurement channel being a channel separate from the medication inhalation channel, i.e., by the flow sensor not being arranged in the medication inhalation channel, an obstruction of the flow sensor by components of the substance to be inhaled can be prevented. Hence, the risk of falsified measurements is reduced. In addition, it is at least partially prevented that components of the substance to be inhaled adhere to the flow sensor. Accordingly, a reduction of the substance provided to the individual can be avoided.

The inhaler measurement device may be a portable inhaler measurement device and/or the puff receiving end may be adapted to be coupled to the mouthpiece of a portable inhaler. Accordingly, the inhaler measurement device may be a portable inhaler measurement device, wherein the puff receiving end is adapted to be coupled to a mouthpiece of a portable inhaler. For example, the inhaler measurement device may be adapted to be coupled to the mouthpiece of a portable asthma inhaler. The term "portable" may refer to the aspect that no additional electric and/or fluid connections are required to operate the respective device, i.e., the inhaler measurement device and/or the inhaler. Further, the term "portable" may refer to the aspect that the respective device, i.e., the inhaler measurement device and/or the inhaler, completely fits into a sphere with a diameter of 15 cm. By the inhaler measurement device being a portable inhaler measurement device, and particularly by the puff receiving end being adapted to be coupled to the mouthpiece of a portable inhaler, the use of the inhaler measurement device under different circumstances is facilitated. For example, measurements can be taken while traveling, during sports activity, and/or in emergency situations. This allows more comprehensive measurement results to be obtained. Nevertheless, it is understood that the inhaler measurement device may alternatively be integrally formed with a portable inhaler.

The flow sensor may be adapted to measure the differential pressure, the volume flow and/or the mass flow. Particularly, the flow sensor may be adapted to measure the differential pressure, the volume flow, and/or the mass flow between the air inlet and the air outlet. In other words, the flow sensor may be adapted to measure the differential pressure, the volume flow, and/or the mass flow within the flow measurement channel. By being adapted to measure the differential pressure, the volume flow, and/or the mass flow, the flow measurement channel may allow for more accurate information on an individual's inhalation behavior.

Further, the flow measurement channel may be arranged such that the puff of spray does not contact the flow sensor. For example, this may be achieved by a respective positioning of the air inlet and the air outlet. Particularly, the air inlet may be located at a position where no contact with a puff of spray is possible. By the flow measurement channel being arranged such that the puff of spray does not contact the flow sensor an obstruction of the flow sensor by components of the substance to be inhaled can be reliably prevented. Thus, the risk of falsified measurements is reduced. In addition, by preventing components of the to-be-inhaled substance from adhering to the flow sensor, the subsequent reduction of the substance provided to the individual can be avoided.

The air outlet of the flow measurement channel may open into the medication inhalation channel, wherein preferably the air outlet of the flow measurement channel opens into the medication inhalation channel in a range of 1 mm to 30 mm prior to the inhalation end, and preferably 2 mm to 25 mm prior to the inhalation end. For example, the air outlet may be an opening being formed in a sidewall of the medication inhalation channel. By the air outlet of the flow measurement channel opening into the medication inhalation channel it can be achieved that the individual inhales only at the inhalation end, i.e., at a single channel, and not at two separate channels. This allows the inhalation behavior of the individual to be accurately monitored. In particular, the risk that the flow measurement channel is obstructed or even closed, e.g., by an individual's tongue, is reduced. Furthermore, the air outlet of the flow measurement channel opening into the medication inhalation channel in a range of 1 mm to 30 mm prior to the inhalation end can on the one hand prevent the air outlet from being impaired by the individual and on the other hand prevent the air flowing out of the air outlet from causing strong turbulence in the puff of spray. By reducing turbulences, a more complete delivery of the puff of spray to the individual can be achieved. One reason for this may be that less turbulence results in less deposition of a puff of spray in the medication inhalation channel.

The air outlet of the flow measurement channel may open into the medication inhalation channel such that the air flow of the flow measurement channel is introduced into the medication inhalation channel substantially parallel to the medication inhalation channel. In other words, the medication inhalation channel and the flow measurement channel may be parallel prior to being merged with each other. This configuration allows to prevent the air flowing out of the air outlet from causing significant turbulences in the puff of spray or at least to minimize the turbulences compared to the case when the flow measurement channel opens into the medication inhalation channel such that an air flow of the flow measurement channel is introduced into the medication inhalation channel perpendicular to the medication inhalation channel. By reducing turbulences, a more complete delivery of the puff of spray to the individual can be achieved. One reason for this may be that less turbulence results in less deposition of a puff of spray in the medication inhalation channel.

The air inlet may be arranged to receive ambient air, wherein preferably the air inlet is arranged at a portion of the housing which is opposite to the inhalation end and/or adjacent to the puff receiving end. By the air inlet being arranged to receive ambient air, an obstruction of the flow sensor by components of the to-be-inhaled substance can be reliably prevented. Thus, the risk of falsified measurements is reduced. In addition, by preventing components of the to-be inhaled substance from adhering to the flow sensor, the subsequent reduction of the substance provided to the individual can be avoided. The air inlet may comprise a filter to protect the flow measurement channel against dust or other particles.

The flow measurement channel may have a diameter which lies in a range from 1 mm to 5 mm, preferably from 1.5 mm to 2.5 mm, further preferably from 1.7 mm to 2.1 mm, and most preferably from 1.8 mm to 2.0 mm. These diameters have been shown to provide accurate measurements of the inhalation behavior.

The medication inhalation channel may have a central axis which is substantially straight-line, wherein the length of the medication inhalation channel preferably lies in a range from 0.5 cm to 10 cm, further preferably from 2 cm to 6 cm, and most preferably from 3 cm to 5 cm. Preferably the complete medication inhalation channel is substantially straight-line. As a result of the straight-line central axis and particularly by the complete medication inhalation channel being straight-line an improved delivery of the puff of spray to the individual can be achieved. Reasons for this may be that curved channels lead to increased deposits and/or turbulences with regards to a puff of spray. Moreover, curved channels are more difficult to produce, e.g., by means of technologies such as injection molding. In addition, the aforementioned length ranges have proven to be advantageous, as they provide sufficient length to perform the required measurements by the puff detection device on the one hand, and on the other hand, they keep adhesion of components of the puff of spray in the medication inhalation channel low.

The inhaler measurement device may further comprise a puff detection device for detecting the puff of spray within the medication inhalation channel. Preferably the puff detection device is arranged between the puff receiving end and the air outlet of the flow measurement channel. By means of the puff detection device and the flow measurement channel the inhaler measurement device according to the present disclosure allows on the one hand a more accurate information on an individual's inhalation behavior and on the other hand a more accurate information on a substance delivery through the inhaler. Thus, it can be precisely determined how much medication was received by an individual.

The puff detection device may comprise an optical sensor and/or a temperature sensor. The optical sensor may comprise a photodetector, a photodiode, a phototransistor, an image sensor, a laser sensor, and/or the like. Further, the optical sensor can be arranged such that it at least partially covers the medication inhalation channel. Hence, a puff of spray can be detected, for example, if the optical sensor detects certain optical properties inside the medication inhalation channel which are characteristic for a puff of spray. Said optical properties may comprise light intensity, wavelength, polarization, reflectivity, transmittance, refractive index, and/or the like. The temperature sensor may comprise a thermocouple, a resistance temperature detector, a thermistor, a pyroelectric sensor, and/or the like. Further, the temperature sensor may comprise an infrared sensor. It is understood that said infrared sensor may be also considered as an optical sensor. The temperature sensor may rely on the aspect that a puff of spray is regularly colder than the ambient air. One reason for this is that expansion from a cartridge of an inhaler results in cooling of the dispensed substance. This temperature difference of the puff of spray compared to the ambient temperature is detected by the temperature sensor when the puff of spray moves past it. Both the optical and the temperature sensors have proven to be advantageous in that they allow for a high precision and/or high reliability. Accordingly, particularly precise and/or reliable measurements can be achieved. Additionally, the temperature sensors have proven to be advantageous in that they are less susceptible to deposits of medication. Accordingly, this results in even higher reliability measurements. Furthermore, infrared range optical sensors have proven to be advantageous as they can transmit directly through common medication inhalation channel materials, such as acrylonitrile butadiene styrene (ABS) and polycarbonate (PC), which avoids complex integration challenges such as clear transparent windows, and keeps the baseline signal (i.e., the signal with no puff of spray) relatively low. Accordingly, this makes the puff of spray easier to detect, as the difference between the baseline signal and the detected signal from a puff of spray is greater.

It is to be noted that a puff detection by means of the flow sensor is possible as well. Hence, the additional puff detection device may serve to increase the likelihood and/or reliability of puff detection. In other words, the flow sensor may be adapted to detect a puff of spray. Thus, the flow sensor may be adapted to measure the differential pressure, the volume flow, the mass flow, and/or to detect a puff of spray.

Preferably the optical sensor and/or the temperature sensor are arranged adjacent to the medication inhalation channel. Further preferably the optical sensor and/or the temperature sensor are separated from the medication inhalation channel via a cover preventing contact of the optical sensor and/or the temperature sensor with the puff of spray. For example, a sidewall of the medication inhalation channel may have a recess in which the optical sensor and/or the temperature sensor are arranged. The choice of material for the cover depends on the properties of the sensor. For example, for a visible light range optical sensor, a clear transparent cover would be preferred, as that would ensure maximum transmission of the light into the medication inhalation channel. For a temperature sensor, a cover that has a high thermal conductivity would be preferred, as that would ensure maximum transmission of the temperature from the medication inhalation channel to the sensor. For an infrared range optical sensor, a cover that has a high transmittance of IR radiation is preferred, as that would ensure maximum transmission of the light into the medication inhalation channel. By being arranged adjacent to the medication inhalation channel the optical sensor and/or the temperature sensor may precisely monitor the inside of the medication inhalation channel so that a precise detection of a puff of spray can be achieved. Furthermore, the cover can avoid deposits and/or protect the optical sensor and/or the temperature sensor.

The optical sensor may comprise an optical proximity sensor, wherein the optical proximity sensor preferably comprises a photoemitter and a photodetector. For example, the proximity sensor may detect a puff of spray as follows. For a visible light range optical proximity sensor, if there is no puff of spray in the medication inhalation channel, the proximity sensor outputs a first signal representing a first distance being measured from the proximity sensor to a sidewall of the medication inhalation channel being opposite to the proximity sensor. For an infrared range optical proximity sensor, if there is no puff of spray in the medication inhalation channel, and the medication inhalation channel material is assumed to be IR-transparent, the proximity sensor outputs a first signal representing the absence of any objects within a detectable range. In both cases, when a puff of spray is in the medication inhalation channel, the proximity sensor outputs a second signal representing a second distance being measured from the proximity sensor to the puff of spray. It is understood that the second distance is less than the first distance. In even further detail, the photoemitter may emit a light signal (i.e., either from a laser-based device with a small beam angle, or from an LED-based device with a large beam angle). If the medication inhalation channel is empty, and the medication channel has a low transmittance of incident light at the wavelength of the photoemitter, the light signal is reflected by an inside surface of the medication inhalation channel and the photodetector outputs the first signal based on the detected reflected light. If the medication channel is empty, and the medication channel has a high transmittance of incident light at the wavelength of the photoemitter, the light signal is transmitted through the medication inhalation channel and the photodetector outputs the first signal based on the absence of any light. However, if a puff of spray is in the medication inhalation channel, the light signal is scattered and/or reflected by the puff of spray. Accordingly, the photodetector detects the reflected and/or scattered light and outputs the second signal. Optical proximity sensors have proven to be advantageous, as they allow the acquisition of several relevant measured variables and permit high accuracy.

A detection area of the optical sensor and/or the temperature sensor may cover an axis extending from the sensor/s substantially perpendicular towards a central axis of the medication inhalation channel. It has shown that a puff of spray within the medication inhalation channel regularly intersects with the central axis of the medication inhalation channel. Hence, with the optical sensor and/or the temperature sensor covering said axis, a particularly reliable detection of a puff of spray can be achieved.

The puff detection device may be configured to detect the velocity, the size, and/or the density of the puff of spray moving from the puff receiving end to the inhalation end. For example, the velocity may be detected by optical measurements, whereas other solutions are also conceivable. In particular, the velocity may be roughly estimated from the duration of the signal detected by the optical sensor, since the length of the medication inhalation channel is known. By measuring the width of the pulse detected from the puff of spray and cross-referencing it with the length of the medication inhalation channel, the approximate velocity of the puff of spray can be determined. Additionally, the velocity may also be detected using Doppler velocimetry. In this case, the velocity can be determined based on the Doppler effect principle, which states that the frequency of light changes when the source or the observer is in motion relative to the other. By comparing the frequency incident to the frequency reflected, the velocity can be precisely calculated. Moreover, the size of the puff of spray may be detected in different ways. For example, in case of an optical sensor, e.g., said proximity sensor, a small puff of spray may be associated with large distances being measured towards the puff of spray. Further exemplarily, in case of a temperature sensor a large puff of spray may be associated with lower temperatures. Furthermore, the density may be for example detected by optical measurements, e.g., by measuring the transmittance of the puff of spray. By the puff detection device being configured to detect the velocity, the size, and/or the density of the puff of spray a more accurate information on a substance delivery by the inhaler can be obtained.

The puff detection device may also be configured to detect the start point and/or the end point of the puff of spray moving from the puff receiving end to the inhalation end. Thereby, more accurate information on a substance delivery by the inhaler can be obtained.

Moreover, the inhaler measurement device may further comprise a processing device being connected to the puff detection device and/or the flow sensor, wherein the processing device may be configured to calculate: if a puff of spray was ejected by the portable inhaler, when a puff of spray was ejected by the portable inhaler, how much spray was ejected by the portable inhaler, how much spray was received by the individual, and/or the intensity and/or duration with which inhalation was performed at the inhalation end. Preferably the processing device comprises a printed circuit board (PCB) and/or a central processing unit (CPU). If and/or when a puff of spray was ejected by the portable inhaler may be calculated based on measurement data from the puff detection device. Further, how much spray was ejected by the portable inhaler may be also calculated based on measurement data from the puff detection device. For example, how much spray was ejected by the portable inhaler may be calculated by the detected size and density of the puff of spray. Moreover, how much spray was received by the individual may be calculated based on measurement data from the puff detection device and the flow sensor. For example, if the puff detection device detected a puff, whereas the flow sensor did not detect a flow then the processing device would calculate that no spray was received by the individual. Furthermore, it is understood that the intensity with which inhalation was performed at the inhalation end may be calculated based on measurement data from the flow sensor. The processing device can enable a local processing of the data, so that no external processing infrastructure is required. In addition, various evaluations can be carried out with the aid of said processing device, wherein said evaluations can be used to improve the uptake of a puff of spray by an individual. Moreover, the processing device may be configured to prepare the measurement data for being transferred to a smartphone for visualization, e.g., in an application.

Furthermore, the inhaler measurement device may further comprise a signaling device being adapted to provide a signal during and/or after inhalation, wherein the signaling device is connected to the processing device, wherein preferably the signaling device provides the signal when: the inhalation is successfully completed, the intensity of inhalation is insufficient, the intensity of inhalation is sufficient, a puff of spray was correctly ejected by the portable inhaler, and/or a puff of spray was incorrectly or not at all ejected by the portable inhaler. It is understood that an instruction to output said signal may be provided by the processing device and/or may be based on calculations thereof. The signaling device may comprise one or more LEDs, a sound generator, and/or a transmitter which may be configured to transfer data, e.g., to a smartphone. Said signaling device can provide an individual with information about his or her inhalation behavior. Accordingly, the individual may adapt the inhalation behavior in order to achieve an improved, e.g., more efficient, uptake of medication.

The inhaler measurement device may further comprise an energy storage device which powers the puff detection device, the processing device, and/or the signaling device, wherein preferably the energy storage is rechargeable, particularly by a USB-port.

The inhaler measurement device may further comprise a contact detector/sensor which is configured to detect a contact of an individual with the inhalation end. The inhaler measurement device may be configured to be activated in case the contact detector detects a contact of an individual with the inhalation end. For example, the contact detector may comprise a capacitive sensor. Furthermore, the contact detector may comprise a metal plate being arranged adjacent to the inhalation end. This metal plate may be arranged to detect a contact of lips with the inhalation end.

Furthermore, the above objective is achieved by a measurement system comprising an inhaler measurement device, as described herein, and a portable inhaler. It is understood that the features and/or advantages described with regards to the inhaler measurement device may also apply for the measurement system.

### 4. Brief description of the accompanying figures

In the following, the accompanying figures are briefly described:
Fig. 1 shows a schematic first inhaler measurement device according to the present invention together with an inhaler in lateral cut view;
Fig. 2 shows a second inhaler measurement device according to the present invention together with an inhaler in perspective view;
Fig. 3 shows the second inhaler measurement device with the inhaler in a lateral cut view;
Fig. 4 shows the second inhaler measurement device without an outer housing portion in a lateral cut view;
Fig. 5 shows the second inhaler measurement device in perspective view, wherein the first inhaler measurement device is laterally cut;
Fig. 6 shows the second inhaler measurement device in a perspective view;
Fig. 7 shows the second inhaler measurement device without an outer housing portion in a perspective view;
Fig. 8 shows a third inhaler measurement device according to the present invention without an outer housing portion in a lateral cut view, and
Fig. 9 shows a fourth inhaler measurement device according to the present invention without an outer housing portion in a lateral cut view.

### 5. Detailed description of the figures

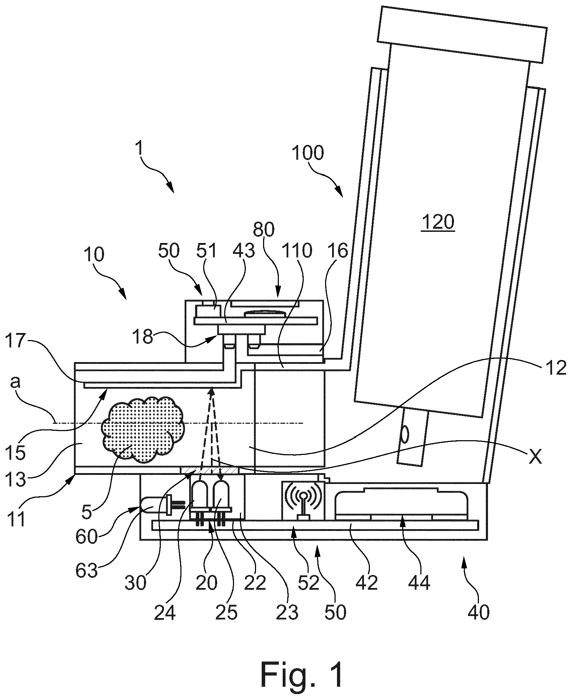

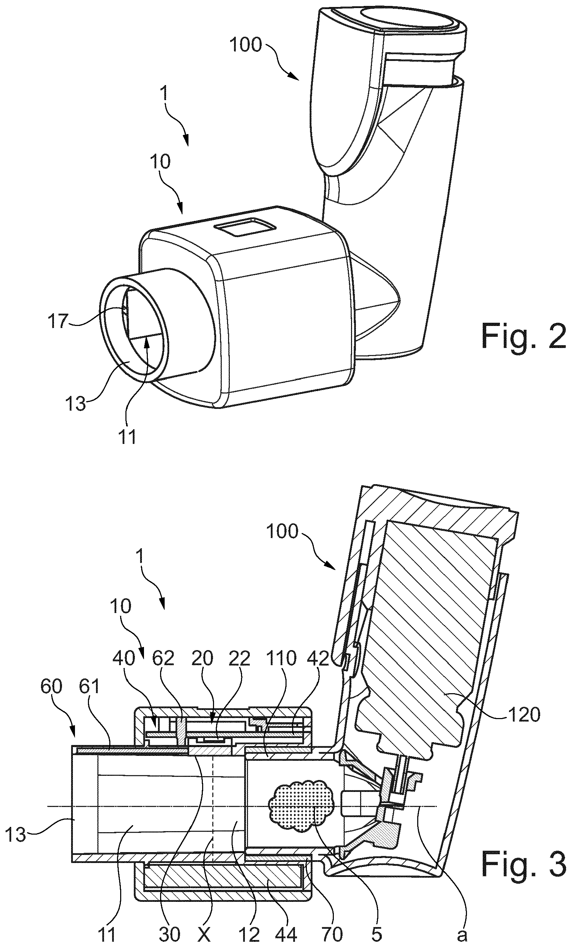

**Fig. 1** shows a different inhaler measurement device 1 than **Figs. 2 to 7****,** **Fig. 8****,** and **Fig. 9****.** However, apart from the differences described, the features described for the inhaler measurement device 1 of the first embodiment may also apply to the inhaler measurement device 1 of the second embodiment, the third embodiment, and/or the fourth embodiment.

In detail, **Fig. 1** shows a portable inhaler measurement device 1, being coupled with a portable inhaler 100. The inhaler measurement device 1 comprises a housing 10 having a medication inhalation channel 11. The medication inhalation channel 11 has a puff receiving end 12 being configured to receive a puff of spray 5 from the portable inhaler 100 and an inhalation end 13 being adapted to provide the puff of spray 5 to an individual. Said puff receiving end 12 is coupled to a mouthpiece 110 of the portable inhaler 100. Moreover, said medication inhalation channel 11 has a central axis a which is straight-line. Particularly, the complete medication inhalation channel 11 is straight-line.

Further, as depicted in **Fig. 1**, the housing 10 comprises a flow measurement channel 15, wherein the flow measurement channel 15 has an air inlet 16, an air outlet 17, and a flow sensor 18 arranged between the air inlet 16 and the air outlet 17. The flow measurement channel 15 is arranged such that the puff of spray 5 does not contact the flow sensor 18. In particular the air inlet 16 is arranged to receive ambient air, wherein the air inlet 16 is arranged at a portion of the housing 10 which is opposite to the inhalation end 13. Further, the air outlet 17 of the flow measurement channel 15 opens into the medication inhalation channel 11. In more detail, the air outlet 17 of the flow measurement channel 15 opens into the medication inhalation channel 11 such that an air flow of the flow measurement channel 15 is introduced into the medication inhalation channel 11 substantially parallel to the medication inhalation channel 11.

F**ig. 1** also shows that the inhaler measurement device 1 further comprises a puff detection device 20 for detecting the puff of spray 5 within the medication inhalation channel 11. The puff detection device 20 is arranged between the puff receiving end 12 and the air outlet 17 of the flow measurement channel 15. The puff detection device 20 comprises an optical sensor 22 being arranged adjacent to the medication inhalation channel 11, wherein the optical sensor 22 is separated from the medication inhalation channel 11 via a transparent cover preventing contact of the optical sensor 22 with the puff of spray 5. In further detail, the optical sensor 22 comprises an optical proximity sensor 23, wherein the optical proximity sensor comprises a photoemitter 24, emitting light in the visible spectrum, and a photodetector 25, optimized for detection in the visible spectrum. Fig. 1 schematically shows the photoemitter 24 emitting a visible light signal and the photodetector 25 detecting the reflected visible light signal from the flow measurement channel 15. Note that Fig. 1 depicts the particular configuration where the light is in the visible range, and the flow measurement channel 15 is not made of a material transparent to visible light. Moreover, a detection area of the optical sensor 22 covers an axis x extending from the sensor substantially perpendicular towards a central axis a of the medication inhalation channel 11. It is understood that the puff detection device 20 may detect the start point and the end point of the puff of spray 5 moving from the puff receiving end 12 to the inhalation end 13, wherein other measurements are also conceivable in dependence of the specific sensor configuration.

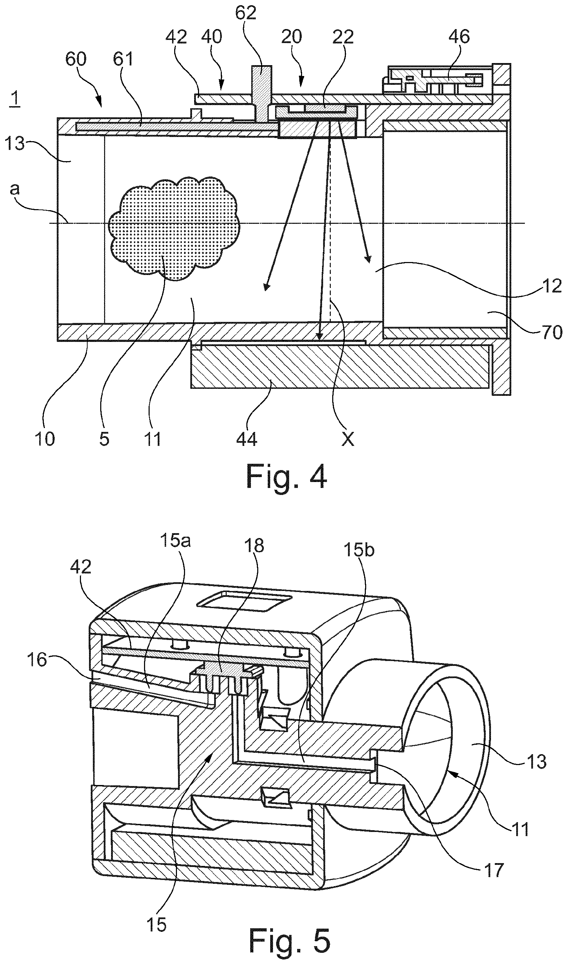

Further, as depicted in **Fig. 1**, the inhaler measurement device 1 further comprises a processing device 40. As depicted in detail in **Fig. 3** and **Fig. 5** for the second inhaler measurement device, the processing device 40 comprises a PCB 42 being connected to the puff detection device 20 and the flow sensor 18. With regards to the first inhaler measurement device 1 of Fig. 1 it is to be noted that the PCB 42 to which the puff detection device 20 is attached may be structurally and/or electrically connected to the further PCB 43 which is associated with the flow sensor 18.

Moreover, **Fig. 1** depicts that the inhaler measurement device 1 further comprises a signaling device 50 being adapted to provide a signal during and/or after inhalation. The signaling device 50 comprises an LED 51 and a transmitter 52. For example, the transmitter 52 may transmit a signal to a smartphone. The signaling device 50 is connected to the processing device 40, i.e., to the PCBs 42 and 43. Even further, the inhaler measurement device 1 comprises a contact detector 60 which is configured to detect a contact of an individual with the inhalation end 13. Said contact detector 60 comprises an optical proximity sensor 63.

**Fig. 2** illustrates for the second inhaler measurement device 1 that the air outlet 17 opens into the medication inhalation channel 11 prior to the inhalation end 13. Moreover, it is depicted that the portable inhaler measurement device 1 is coupled with a portable inhaler 100.

**Fig. 3** depicts that the second inhaler measurement device 1 comprises a contact detector 60 which is configured to detect a contact of an individual with the inhalation end 13. Said contact detector 60 comprises a metal plate 61 being arranged adjacent to the inhalation end 13. This metal plate 61 is arranged to detect a contact of lips with the inhalation end 13. The metal plate 61 is connected to the PCB 42 via a metal pin 62. The metal pin 62 may be any other electrically conductive element. Moreover, it is to be noted that the metal plate 61 may be replaced by a PCB which provides the respective functionality. Further, the second inhaler measurement device 1 comprises an adapter 70 which allows to couple the inhaler measurement device 1 with different portable inhalers 100.

It is to be noted that the configuration of the inhaler measurement device 1 in **Fig. 3** may comprise a temperature sensor instead of the optical sensor 22.

**Fig. 4** shows that a USB-port 46 is attached on the PCB 42. Said USB-port 46 may serve to charge the battery 44. Moreover, by means of arrows being directed towards the middle axis of the medication inhalation channel the detection area of the optical sensor 22 is illustrated.

**Fig. 5** shows that the flow measurement channel 15 comprises a first channel portion 15a being associated with the air inlet 16. Further, the flow measurement channel 15 comprises a second channel portion 15b being associated with the air outlet 17.

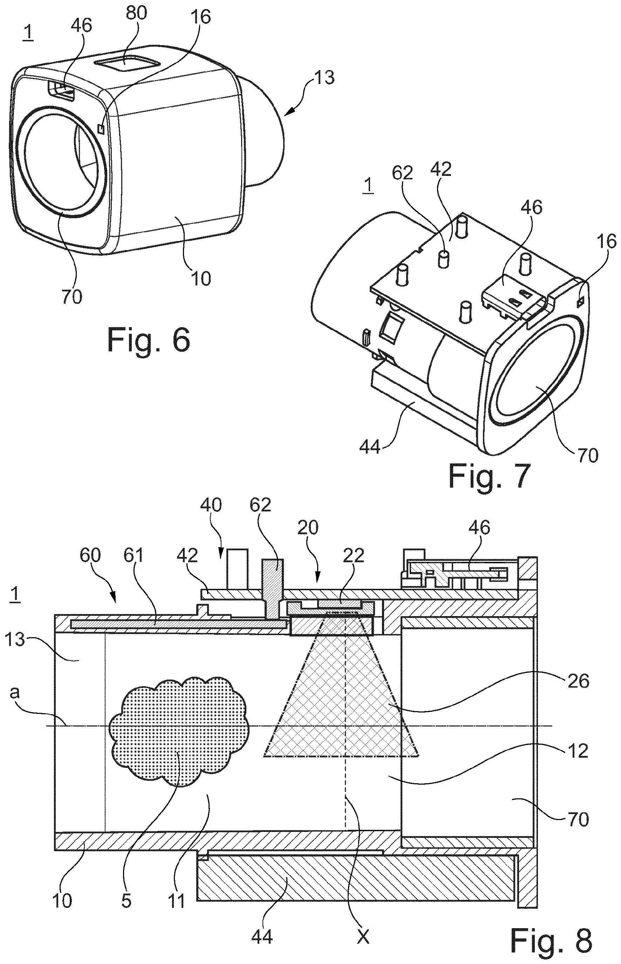

**Fig. 6** shows that the air inlet 16 is arranged to receive ambient air, wherein the air inlet 16 is arranged at a portion of the housing which is opposite to the inhalation end 13. Moreover, Fig. 6 shows that the inhaler measurement device 1 comprises an activation button 80.

**Fig. 7** shows the inhaler measurement device 1 without an outer housing sleeve so that four attachment pins for the attachment of the PCB 42 are visible. Another preferred embodiment comprises three attachment pins.

Further, the inhaler measurement device 1 depicted in **Fig. 8** also comprises a puff detection device 20 for detecting the puff of spray 5 within the medication inhalation channel 11. The puff detection device 20 comprises an optical sensor 22, wherein the optical sensor 22 is an infrared (IR) sensor. Particularly, the IR sensor 22 is an infrared range optical sensor. The IR sensor 22 has a detectable range 26 that is schematically illustrated. If there is no puff of spray 5 in the detectable range 26, then the IR sensor 22 outputs a first signal representing the absence of any objects within a detectable range 26. However, when a puff of spray 5 is in the detectable range 26, the IR sensor 22 outputs a second signal representing the presence of an object within a detectable range 26. As further depicted, the inhaler measurement device 1 has no transparent cover like the other depicted inhaler measurement devices. This is as the IR sensor 22 can transmit directly through the medication inhalation channel material. Nevertheless, it is understood that in a different embodiment a transparent cover may be provided.

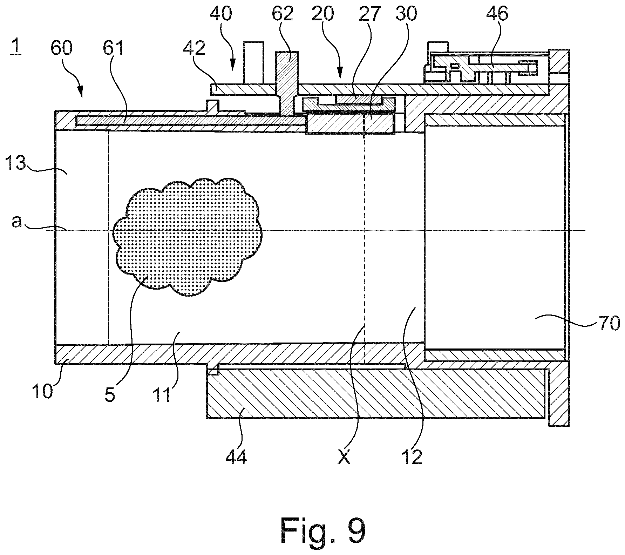

Moreover, the inhaler measurement device 1 depicted in **Fig. 9** also comprises a puff detection device 20 for detecting the puff of spray 5 within the medication inhalation channel 11. The puff detection device 20 comprises a temperature sensor 27. The temperature sensor 27 is configured to detect the temperature difference of the puff of spray 5 compared to the ambient temperature when the puff of spray moves past the temperature sensor 27. Furthermore, the inhaler measurement device 1 has a cover 30 that has a high thermal conductivity, as that ensures maximum transmission of the temperature from the medication inhalation channel 11 to the temperature sensor 27.

It is understood by the skilled person that **Figs. 1 to 3** show a measurement system comprising an inhaler measurement device 1 as described herein and a portable inhaler 100.

### List of reference signs

- 1: inhaler measurement device
- 5: puff of spray
- 10: housing
- 11: medication inhalation channel
- 12: puff receiving end
- 13: inhalation end
- 15: flow measurement channel
- 16: air inlet
- 17: air outlet
- 18: flow sensor
- 20: puff detection device
- 22: optical sensor
- 23: proximity sensor
- 24: photoemitter
- 25: photodetector
- 26: detectable range of the IR-sensor
- 27: temperature sensor30 transparent cover
- 40: processing device
- 42: PCB
- 43: further PCB
- 44: battery
- 46: USB port
- 50: signaling device
- 51: LED
- 52: transmitter
- 60: contact detector
- 61: metal plate
- 62: metal pin
- 63: optical proximity sensor
- 70: adapter
- 80: activation button
- 100: portable inhaler
- 110: mouthpiece
- 120: cartridge
- x: axis
- a: central axis

## Claims

1. An inhaler measurement device (1), in particular an inhaler flow measurement device, for a portable inhaler (100), wherein the inhaler measurement device (1) comprises
a housing (10) having a medication inhalation channel (11), wherein the medication inhalation channel (11) has a puff receiving end (12) being configured to receive a puff of spray (5) from the portable inhaler (100) and an inhalation end (13) being adapted to provide the puff of spray (5) to an individual, and wherein the housing (10) further comprises a flow measurement channel (15), wherein the flow measurement channel (15) has an air inlet (16), an air outlet (17), and a flow sensor (18) arranged between the air inlet (16) and the air outlet (17).

2. The inhaler measurement device (1) according to the preceding claim, wherein the inhaler measurement device (1) is a portable inhaler measurement device (1) and/or the puff receiving end (12) is adapted to be coupled to a mouthpiece of a portable inhaler (100).

3. The inhaler measurement device (1) according to any one of the preceding claims, wherein the flow sensor (18) is adapted to measure the differential pressure, the volume flow and/or the mass flow.

4. The inhaler measurement device (1) according to any one of the preceding claims, wherein the flow measurement channel (15) is arranged such that the puff of spray (5) does not contact the flow sensor (18).

5. The inhaler measurement device (1) according to any one of the preceding claims, wherein the air outlet (17) of the flow measurement channel (15) opens into the medication inhalation channel (11), wherein preferably the air outlet (17) of the flow measurement channel (15) opens into the medication inhalation channel (11) in a range of 1 mm to 30 mm prior to the inhalation end (13).

6. The inhaler measurement device (1) according to the preceding claim, wherein the air outlet (17) of the flow measurement channel (15) opens into the medication inhalation channel (11) such that an air flow of the flow measurement channel (15) is introduced into the medication inhalation channel (11) substantially parallel to the medication inhalation channel (11).

7. The inhaler measurement device (1) according to any one of the preceding claims, wherein the air inlet (16) is arranged to receive ambient air, wherein preferably the air inlet (16) is arranged at a portion of the housing (10) which is opposite to the inhalation end (13) and/or adjacent to the puff receiving end (12).

8. The inhaler measurement device (1) according to any one of the preceding claims, wherein the flow measurement channel (15) has a diameter which lies in a range from 1 mm to 5 mm, preferably from 1.5 mm to 2.5 mm, further preferably from 1.7 mm to 2.1 mm, and most preferably from 1.8 mm to 2.0 mm.

9. The inhaler measurement device (1) according to any one of the preceding claims, wherein the medication inhalation channel (11) has a central axis (a) which is substantially straight-line, wherein the length of the medication inhalation channel (11) preferably lies in a range from 0.5 cm to 10 cm, further preferably from 2 cm to 6 cm, and most preferably from 3 cm to 5 cm.

10. The inhaler measurement device (1) according to any one of the preceding claims, wherein the inhaler measurement device (1) further comprises
a puff detection device (20) for detecting the puff of spray (5) within the medication inhalation channel (11), wherein preferably the puff detection device (20) is arranged between the puff receiving end (12) and the air outlet (17) of the flow measurement channel (15).

11. The inhaler measurement device (1) according to the preceding claim, wherein the puff detection device (20) comprises an optical sensor (22) and/or a temperature sensor (27), wherein preferably the optical sensor (22) and/or the temperature sensor (27) are arranged adjacent to the medication inhalation channel (11), wherein further preferably the optical sensor (22) and/or the temperature sensor (27) are separated from the medication inhalation channel (11) via a cover preventing contact of the optical sensor (22) and/or the temperature sensor (27) with the puff of spray (5).

12. The inhaler measurement device (1) according to any one of the preceding claims 10 or 11, wherein the puff detection device (20) is configured to detect the velocity, the size, and/or the density of the puff of spray (5) moving from the puff receiving end (12) to the inhalation end (13).

13. The inhaler measurement device (1) according to any one of the preceding claims, wherein the inhaler measurement device (1) further comprises a processing device (40) being connected to the puff detection device (20) and/or the flow sensor (18), wherein the processing device (40) is configured to calculate: if a puff of spray (5) was ejected by the portable inhaler (100), when a puff of spray (5) was ejected by the portable inhaler (100), how much spray was ejected by the portable inhaler (100), how much spray was received by the individual, and/or the intensity and/or duration with which inhalation was performed at the inhalation end (13).

14. The inhaler measurement device (1) according to the preceding claim, wherein the inhaler measurement device (1) further comprises a signaling device (50) being adapted to provide a signal during and/or after inhalation, wherein the signaling device (50) is connected to the processing device, wherein preferably the signaling device (50) provides the signal when: the inhalation is successfully completed, the intensity of inhalation is insufficient, the intensity of inhalation is sufficient, a puff of spray (5) was correctly ejected by the portable inhaler (100), and/or a puff of spray (5) was incorrectly or not at all ejected by the portable inhaler (100).

15. A measurement system comprising an inhaler measurement device (1) according to any one of the preceding claims and a portable inhaler (100).
